# FASCICULE DE BREVET EUROPEEN

(11) **EP 0 375 584 B1**
(45) Date de publication et mention de la délivrance du brevet: **14.04.1993**
(21) Numéro de dépôt: 89480168.7
(22) Date de dépôt: 24.10.1989
(51) Int. Cl.: A61M 5/20, A61M 5/46

(54) **Dispositif d'injection à usage médical et vétérinaire évitant les contaminations**
Injektionsvorrichtung für medizinischen und tierärztlichen Gebrauch, die Infektionen verhindert
Injection device for medical and veterinary use preventing contaminations

(30) Priorité: 21.11.1988 FR 8815538
(43) Date de publication de la demande: 27.06.1990
(73) Titulaire: Denance, Raymond, F-06270 Villeneuve Loubet (FR)
(72) Inventeur: Denance, Raymond, F-06270 Villeneuve Loubet (FR)
(74) Mandataire: Hautier, Jean-Louis

(56) Documents cités:
- FR-A- 2 390 175
- FR-A- 2 524 321
- FR-A- 2 567 760
- US-A- 3 790 048
- US-A- 4 198 975

## Description

L'invention a pour objet un dispositif d'injection à usage médical et vétérinaire dont la mire stabilisatrice est à usage unique, ladite mire actionne un moyen faisant office de clapet au niveau de l'aiguille.

Cet appareil est destiné à réaliser des injections intradermiques, sous-cutanées ou intramusculaires à usage médical ou vétérinaire et plus particulièrement en mésothérapie, hydropuncture, vaccinations ou tests allergologiques.

L'état de la technique peut être défini par le brevet FR-A-2.524.321 du même auteur. Ce brevet décrit un dispositif d'injection "manuelle ou mécanique" à usage médical et vétérinaire. Ce brevet décrit un dispositif d'injection qui comporte : une crosse bâti ayant la forme d'un révolver, une seringue d'injection amovible pourvue d'un piston de seringue, d'une aiguille d'injection unique ou d'un multi-injecteur portant plusieurs aiguilles d'injection, un berceau de seringue rendant solidaire cette seringue à la crosse bâti, des moyens pour positionner l'impact et prédéterminer le degré de pénétration de l'aiguille ou des aiguilles, des moyens de transmission mécanique comprenant un cliquet et un barillet transmettant la poussée excercée pour déclencher la pénétration de la ou des aiguilles et l'injection du liquide.

L'état de la technique peut également être défini par des brevets tels que le brevet HALLER US-A.4.198.975 et le brevet LUCIANO US-A.3.790.048.

Ces brevets décrivent notamment des pistolets pour faire des piqûres, un des pistolets est électrique, il s'agit de celui du brevet LUCIANO,

Enfin, l'état de la technique est aussi défini par le brevet FR.B2.2.390.175 qui décrit un procédé de traitement mésothérapique et son dispositif d'injection formant micro injecteur automatique. Ce dispositif comprend toutes les caractéristiques du préambule de la revendication 1.

Les problèmes, développés par rapport aux maladies contagieuses à développement rapide, impliquent une révision de ce type de pistolet à usage médical et vétérinaire. A usage médical, ce type de pistolet est utilisé notamment en mésothérapie. Il est important que toutes les parties du dispositif d'injection en contact avec le patient à traiter, puissent être à usage unique.

Un autre problème se pose sur le plan pratique avec ce type de dispositif d'injection tel que défini par l'état de la technique. La pression excercée par le piston au niveau de la seringue concerve une certaine inertie due à la surpession, ce qui implique qu'à chaque sortie de l'aiguille de la peau, il y a une perte de liquide. Des gouttelettes se répandent, ce qui est désagréable pour le patient et pour la personne qui utilise le dispositif d'injection. Ces fuites de liquide représentent également, dans le cas où le liquide injecté est relativement cher, une perte d'argent non négligeable.

Enfin, dans le cas de traitement spécifique tel que le traitement de la cellulite, l'utilisateur du dispositif doit actionner quatre à cinq cent fois la gâchette pour que ledit dispositif injecte le liquide. Après plusieurs traitements, il existe une fatigue quant à l'utilisation des dispositifs mécaniques actuels sur le marché.

L'invention tend à résoudre tous ces problèmes.

Le dispositif, selon l'invention, est un dispositif d'injection électrique comprenant une crosse bâti, une seringue qui repose sur la face supérieure horizontale de la crosse bâti et, est maintenue dans la partie avant par son embout qui repose sur un étrier et dans la partie arrière par la tête du piston de la seringue qui est logée dans un curseur mobile actionné par un moteur électrique qui transmet la poussée pour déclencher la pénétration de l'aiguille et l'injection du liquide, une mire stabilisatrice de la peau qui guide et prédétermine le degré de pénétration de l'aiguille par un règlage.

La mire stabilisatrice est amovible, un moyen de maintien faisant office de ressort assure son maintien dans une vis creuse micrométrique elle-même logée dans un cylindre mobile ; ledit cylindre mobile comportant un doigt mobile qui agit sur un moyen faisant office de clapet disposé au niveau de l'aiguille d'injection.

La tête du piston de la seringue est en contact avec la tête d'un piston situé dans le curseur mobile, le piston est pourvu d'un ressort monté coaxialement à la tige du piston ; ledit ressort est logé dans le curseur mobile, l'ensemble curseur et piston est actionné par le moteur électrique qui agit sur un axe fileté sur lequel est monté le corps du curseur qui forme, à ce niveau, un écrou de manoeuvre.

La tête du piston est en prise par l'écrou de manoeuvre avec un axe fileté dont une extrémité repose sur un palier situé dans la crosse bâti et dont l'autre extrémité se termine par un pignon denté qui vient engrener sur un pignon denté situé à la sortie d'un réducteur disposé dans le prolongement du moteur électrique.

Dans le mode de réalisation électrique et électronique, le moteur peut être pas à pas ; le moteur engrène, par son axe et sa roue dentée, sur une autre roue en prise directe avec l'axe fileté ; sur l'axe fileté est monté un curseur qui est en prise avec la tête du piston de la seringue.

Un commutateur de fonctionnement est disposé dans le prolongement de l'axe de la chambre et du cylindre qui logent la mire stabilisatrice, ce commutateur de fonctionnement assure le déclenchement du fonctionnement en automatique, continu ou simultané, par action de la mire dès que celle-ci est en contact avec la peau ;
- contact marche avant ;
- contact marche arrière ;
- interrupteur avant ;
- interrupteur arrière ;
- contacteur marche/arrêt.

Le dispositif est pourvu d'un signal, qui est un signal sonore, qui est déclenché à chaque injection.

La mire stabilisatrice pénètre par son extrémité, dans un orifice de la partie antérieure de la crosse bâti ; cette extrémité est logée dans une vis micrométrique creuse ; ladite vis micrométrique creuse est filetée sur sa face extérieure de manière à pouvoir se déplacer dans une chambre d'un cylindre mobile prévue à cet effet ; ledit cylindre mobile se termine par un axe ; ledit axe pénètre dans un palier, solidaire de la crosse bâti ; un ressort de rappel est disposé coaxialement audit axe entre la chambre et l'extrémité de son axe ; une molette de réglage, permet de faire tourner l'axe et la chambre ; ladite chambre du cylindre mobile comporte un filetage dans son corps intérieur ; le filetage correspond au filetage de la face extérieure de la vis micrométrique ; en faisant tourner la molette de réglage, l'axe et la chambre tournent tandis que la vis micrométrique se déplace dans ladite chambre du cylindre mobile ; cette molette de réglage permet ainsi de régler la profondeur d'entrée de l'extrémité de la mire et de régler ainsi le degré de pénétration de l'aiguille dans la peau.

La mire stabilisatrice se termine par son extrémité qui est cruciforme et qui pénétre dans le corps du cylindre qui est fendu selon son axe longitudinal et uniquement dans sa partie antérieure tandis qu'un moyen de maintien est mis en place autour du corps du cylindre pour faire office de ressort de fixation.

Le moyen de maintien est un anneau de maintien qui est mis en place autour du corps de la vis micrométrique creuse.

Le dispositif est pourvu d'un moyen faisant office de clapet disposé au niveau de l'aiguille d'injection, ledit clapet étant associé à un moyen faisant office de détecteur de pénétration de l'aiguille dans la peau.

Le corps du raccord pour clapet est disposé entre l'embout de fixation de l'aiguille et l'embout de la seringue où s'emboîte habituellement l'aiguille.

Le corps du raccord pour clapet est muni d'un moyen faisant office de levier qui est actionné par un doigt mobile ; ledit doigt mobile est vissé sur l'extrémité antérieure de la chambre du cylindre mobile ; la vis micrométrique creuse peut également être filetée à ce niveau et comporter une butée d'arrêt ; le doigt mobile est extérieur tandis que son corps est cylindrique pour s'emmancher entre la chambre du cylindre mobile et la vis micrométrique ; ledit corps du raccord pour clapet est donc asservi par un moyen qui fait office de détecteur de pénétration de l'aiguille dans la peau ; dès que l'aiguille est retirée de la peau, le clapet ferme l'orifice du corps du raccord pour clapet ce qui ferme le passage de liquide de la seringue vers l'aiguille par ledit orifice.

Le moyen faisant office de détecteur de pénétration de l'aiguille dans la peau est un levier, ledit levier agit au niveau de la paroi du corps du raccord pour clapet ; ladite paroi est, à ce niveau, légèrement plus mince ; ledit levier agit donc directement sur le clapet qui est monté libre dans la chambre ce qui autorise un basculement qui dégage ledit clapet de l'orifice qui autorise la circulation du liquide entre la seringue et l'aiguille.

La chambre du corps du raccord pour clapet peut comporter des ergots qui permettent de faire entrer en force le clapet dans ladite chambre, mais éviter que celui-ci ne puisse sortir.

Le clapet a une forme en tronc de cône.

Les dessins ci-joints sont donnés à titre d'exemples indicatifs et non limitatifs. Ils représentent un mode de réalisation préféré selon l'invention. Ils permettront de comprendre aisément l'invention.

La figure 1 est une vue en coupe du dispositif d'injection à usage médical et vétérinaire qui met en évidence les principaux organes actifs dudit dispositif. Cette figure comprend une "bulle" qui met en évidence les détails des moyens de fixation de la mire dans le pistolet.

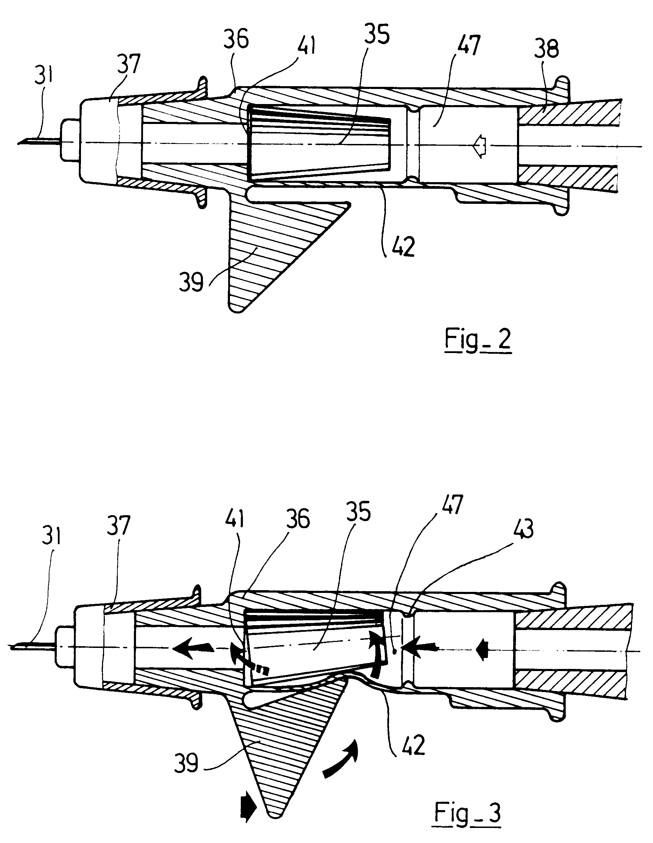

La figure 2 est une vue en coupe du corps du raccord pour clapet en position fermée.

La figure 3 est une vue en coupe mettant en évidence le corps du raccord pour clapet, son clapet mobile et l'action du levier qui agit dans le sens indiqué par les flèches.

La figure 4 est une vue en coupe du dispositif d'injection adapté à une alimentation électrique par piles ou batteries rechargeables ou non.

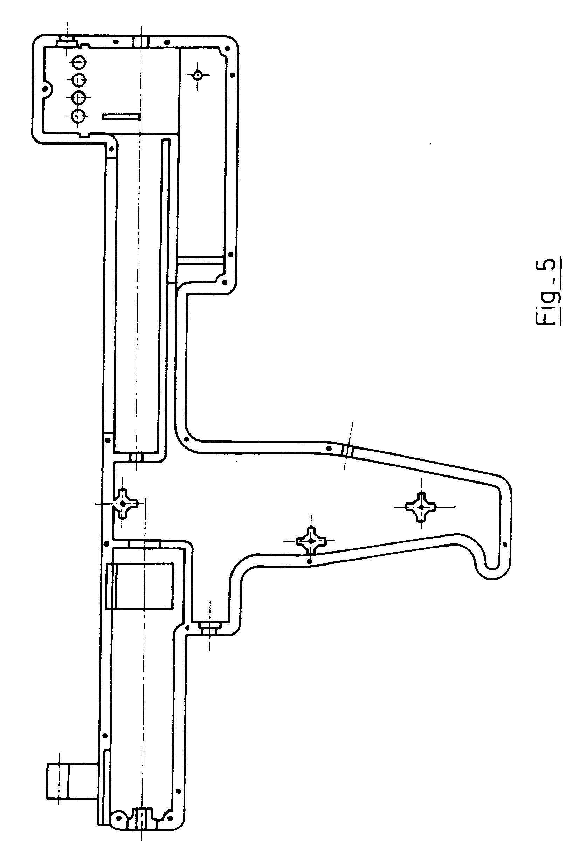

La figure 5 est une vue schématique du dispositif d'injection selon l'invention, selon un mode de réalisation électrique ou électronique.

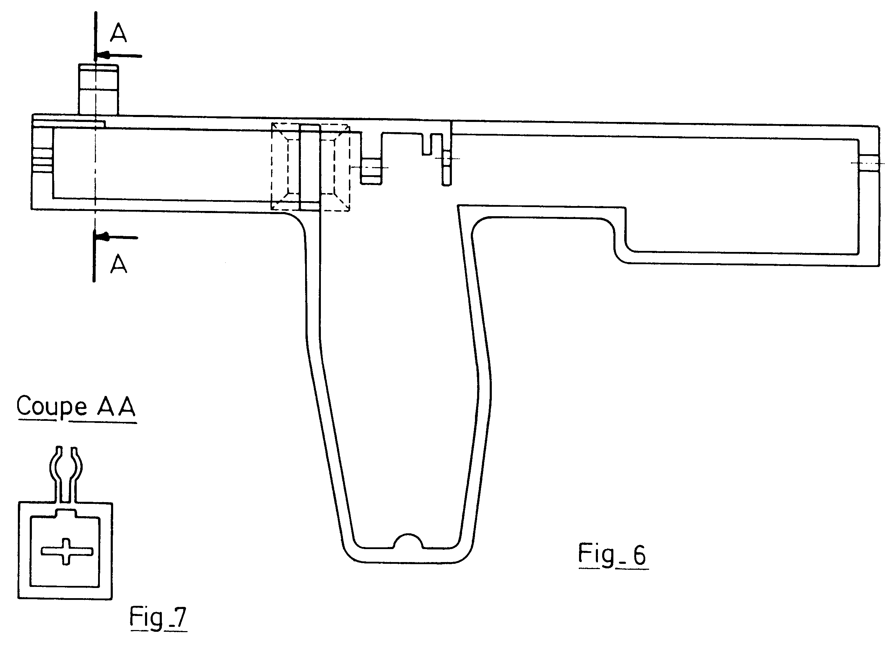

La figure 6 est une vue schématique du dispositif selon l'invention, selon un autre mode de réalisation.

La figure 7 est une vue en coupe, section AA représentée à la figure 6, de la crosse bâti du dispositif mettant en évidence l'étrier et l'entrée cruciforme pour l'extrémité de la mire stabilisatrice. Dans ce mode de réalisation l'ensemble, est monobloc, obtenu par injection.

Le dispositif selon l'invention est un pistolet d'injection électrique. Il comprend une crosse bâti 1 ayant la forme d'un révolver. Dans sa partie supérieure, et perpendiculairement au manche 2 de la crosse bâti 1, est disposé vers l'avant un étrier 3 et vers l'arrière un curseur mobile 6. La seringue 4 est maintenue au niveau de son embout par l'étrier 3 sur laquelle ledit embout repose et vers l'arrière par son piston 5 qui est logé dans un curseur 6. Le piston 5 de la seringue 4 est actionné par un piston 7 logé dans le curseur 6 dont la tige est pourvue coaxialement d'un ressort 8. Le corps du curseur 6 forme un écrou de manoeuvre 45, ledit écrou de manoeuvre 45 du curseur mobile 6 est en prise avec un axe fileté 10 dont une extrémité 11 repose sur un palier 12 situé dans la crosse bâti 1, et dont l'autre extrémité 13 se termine par un pignon denté 14 qui vient engrener sur un autre pignon denté 15 situé à la sortie d'un réducteur disposé dans le prolongement du moteur électrique 17.

Selon un mode de réalisation préféré, le moteur 17 et le réducteur 16 sont disposés dans le manche 2 de la crosse bâti 1.

Les différents contacts de fonctionnement et commutateurs sont les suivants :
- un commutateur de fonctionnement 18 est disposé dans le prolongement de l'axe du cylindre mobile qui loge la mire stabilisatrice, ce commutateur de fonctionnement 18 assure le déclenchement du fonctionnement en automatique, continu ou simultané, par action de la mire dès que celle-ci est en contact avec la peau (ce commutateur de fonctionnement peut être de simples contacts mécaniques ou par exemple un détecteur optique) ;
- 19 est le contact marche avant ;
- 20 est le contact marche arrière ;
- 21, disposé dans le prolongement de la tête 9 du piston 7, est l'interrupteur avant ;
- 22, solidaire du curseur 6, est l'interrupteur arrière ;
- 23 est le contacteur marche/arrêt.

Le dispositif peut être pourvu d'un signal 44, signal sonore ou lumineux.

Dans le mode de réalisation représenté, ledit signal 44 est un signal sonore, il peut être intéressant, pour l'utilisateur, de connaître le nombre d'injections effectuées. Certains dispositifs comportent des compteurs difficiles à surveiller tout en manipulant le dispositif d'injection. D'autres dispositifs comportent des écrans avec affichage par diodes luminescentes qui présentent le même inconvénient qu'un compteur numérique.

Le signal auditif ou lumineux, selon l'invention, présente l'avantage d'habituer le praticien à un rythme, par exemple sonore, qui correspond à une quantité de liquide en millimètres/cube, injecté au patient.

Sans compter, de manière précise, le nombre d'injections, par la simple cadence et le temps passé, l'utilisateur sait s'il a injecté la quantité de liquide suffisante et nécessaire. Le corps du curseur 6 se prolonge au-delà de l'axe de la seringue 4 pour servir de écrou de manoeuvre 45 à l'axe fileté 10.

Comme indiqué dans le préambule de l'invention, la mire stabilisatrice 24 est à usage unique, elle évite ainsi les problèmes dus aux maladies contagieuses.

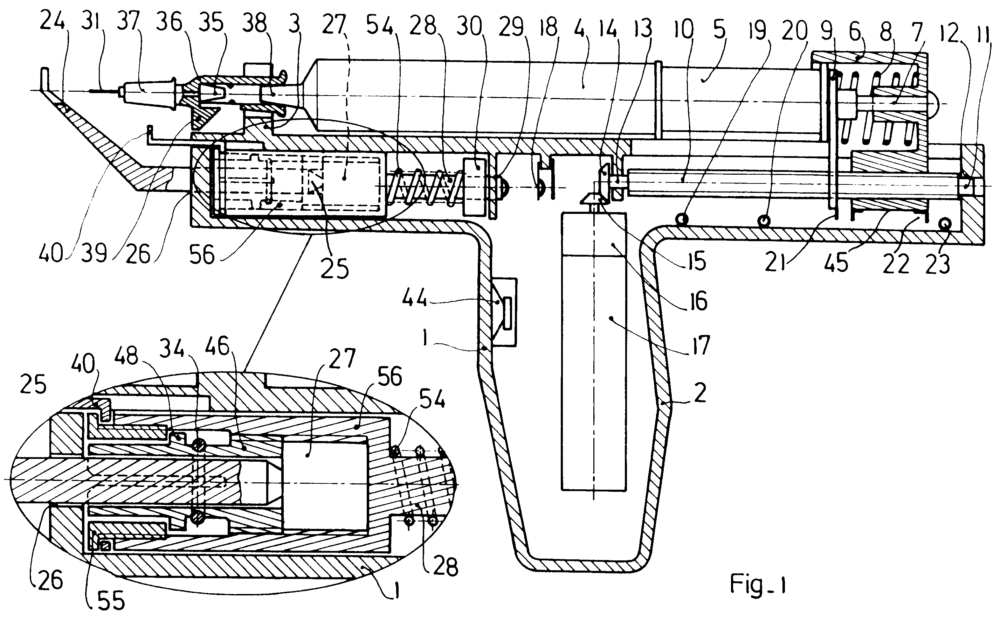

La mire stabilisatrice 24 pénètre par son extrémité 25, dans un orifice 26 de la partie antérieure de la crosse bâti 1. Cette extrémité 25 est logée dans une vis micrométrique 46 creuse dont les parties extérieures sont filetées (voir la figure 1). Ladite vis micrométrique 46 est filetée sur sa face extérieure de manière à pouvoir se déplacer dans la chambre 27 d'un cylindre mobile 56 prévue à cet effet. Ladite chambre 27 du cylindre mobile 56 se termine par un axe 28. Ledit axe 28 pénètre dans un palier 29, solidaire de la crosse bâti. Un ressort de rappel 54 est disposé coaxialement audit axe 28 entre la chambre 27 du cylindre mobile 56 et l'extrémité de son axe 28. Une molette de réglage 30, permet de faire tourner l'axe 28 et la chambre 27 du cylindre mobile 56. Ladite chambre 27 du cylindre mobile 56 comporte un filetage dans son corps intérieur. Le filetage correspond au filetage de la vis micrométrique 46. En faisant tourner la molette de réglage 30, l'axe 28 et la chambre 27 du cylindre mobile 56 tournent tandis que la vis micrométrique 46 se déplace dans ladite chambre 27 du cylindre mobile 56. Cette molette de réglage 30 permet ainsi de régler la profondeur d'entrée de l'extrémité 25 de la mire 24 et de régler ainsi le degré de pénétration de l'aiguille 31 dans la peau. Ladite molette 30 règle ainsi la course de la mire stabilisatrice 24 et de guidage qui fait ainsi office, en même temps, de moyen de réglage de pénétration de l'aiguille 31 dans la peau. L'ensemble cylindre mobile 56 et vis micrométrique creuse 46 est maintenu dans le corps de la crosse bâti par un bouchon fileté creux 55 qui laisse passer l'extrémité 25 de la mire stabilisatrice 24.

La mire stabilisatrice 24 se termine par son extrémité 25 qui est cruciforme et qui pénétre dans le corps du cylindre 46 qui est fendu selon son axe longitudinal et uniquement dans sa partie antérieure tandis qu'un moyen de maintien 34 est mis en place pour assurer la fixation de cette extrémité 25.

Selon le mode de représentation de la figure 1, le moyen de maintien est un anneau de maintien 34 qui est mis en place autour du corps de la vis micrométrique creuse 46.

La mire stabilisatrice 24 est donc amovible, elle peut entrer ou sortir en force dans son logement, elle est donc à usage unique et donc jetable.

Une des caractéristiques de l'invention réside dans le fait que le dispositif d'injection est pourvu d'un moyen 35 faisant office de clapet disposé au niveau de l'aiguille d'injection 4.

Ce dispositif faisant office de clapet évite les problèmes de fuite du liquide d'injection.

Un corps du raccord pour clapet 36 est disposé entre l'embout 37 de fixation de l'aiguille et l'embout 38 de la seringue où s'emboîte habituellement l'aiguille.

Ledit corps du raccord pour clapet 36 est muni d'un moyen faisant office de levier 39 qui est actionné par un doigt mobile 40. Ledit doigt mobile 40 est vissé sur l'extrémité antérieure de la chambre 27 du cylindre mobile 56. A cet effet, la vis micrométrique 46 peut également être filetée à ce niveau et comporter une butée d'arrêt 48. Le doigt mobile 40 est extérieur, tandis que son corps est cylindrique pour s'emmancher entre la chambre 27 du cylindre mobile 56 et la vis micrométrique 46.

Ledit corps du raccord pour clapet 36 est donc asservi par un moyen qui fait office de détecteur de pénétration de l'aiguille 31 dans la peau. Dès que l'aiguille 31 est retirée de la peau, le clapet 35 ferme l'orifice du corps du raccord pour clapet 36 ce qui ferme le passage de liquide de la seringue 4 vers l'aiguille 31 par ledit orifice.

Le moyen faisant office de levier 39 est un détecteur de pénétration de l'aiguille dans la peau. Ledit levier 39 agit directement au niveau de la paroi 42 du corps du raccord pour clapet 36. Ladite paroi 42 est, à ce niveau, légèrement plus mince. Ledit levier 39 agit donc directement sur le clapet 35. Le clapet 35 est dégagé de l'orifice 41 qui autorise la circulation du liquide entre la seringue 4 et l'aiguille 31. La constitution du corps du raccord pour clapet 36 peut être du plastique dont les qualités utilisées sont notamment "la mémoire de plastique" qui reprend sa forme après action du levier 39 au niveau de la paroi souple 42 et ce, de manière à ce que ledit levier 39 puisse déformer ladite paroi et agir, à l'intérieur de la chambre 47, sur le clapet 35. La chambre 47 du corps du raccord pour clapet 36 peut comporter des ergots 43 qui permettent de faire entrer en force le clapet 35 dans ladite chambre 47, mais éviter que celui-ci ne puisse sortir. Ledit clapet 35 est donc monté libre dans la chambre 47.

De manière à pouvoir agir et régler la pression d'injection, la tige du piston se termine vers l'extérieur du piston par un orifice fileté 54 dans lequel vient se loger une vis 55 dont la tête est extérieure au curseur 6. L'action sur la vis 55 permet de régler la pression du ressort 8 qui est logé dans la chambre du curseur 6. Le réglage de la tension du ressort 8 permet de régler la pression d'injection ou poussée.

Selon un mode de réalisation préféré, le clapet 35 est en forme de tronc de cône dont le sommet est orienté vers la seringue 4 et la base forme l'orifice 41 de la chambre 47. Le clapet 35 peut avoir une forme en tronc de cône qui facilite le basculement et le dégagement de l'orifice 41 sur l'action du levier 39.

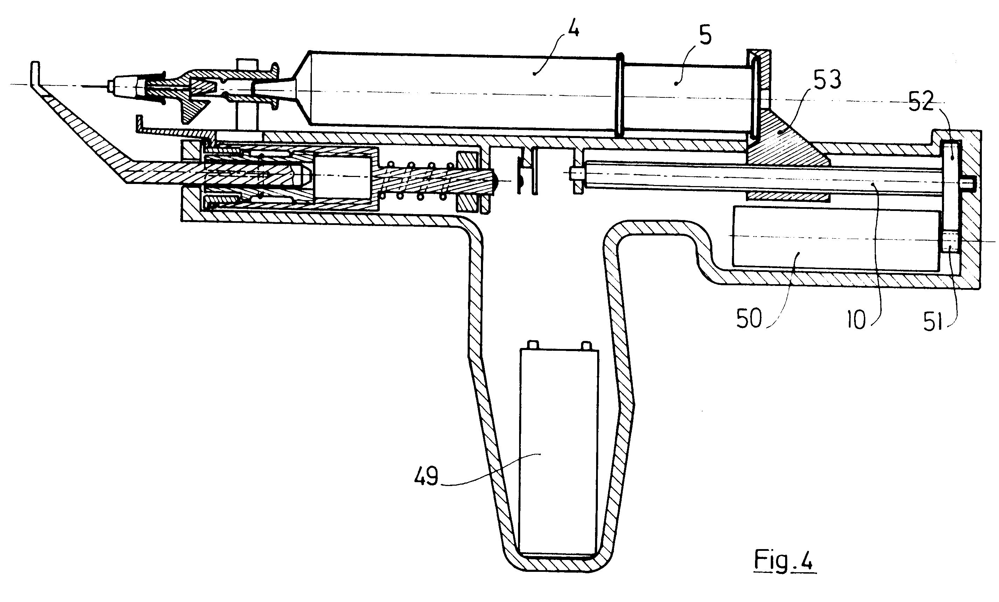

La figure 4 représente un autre mode de réalisation électrique et électronique permettant un choix de programmes pour l'injection en fonction des pathologies à traiter et du choix de l'appareil par le médecin. Le moteur 50 peut, dans ce cas, être pas à pas et le mécanisme de poussée au niveau du piston de la seringue peut être différent. Dans le cas représenté à la figure 4, le moteur 50 engrène, par son axe et sa roue dentée 51, sur une autre roue 52 en prise directe avec l'axe fileté 10. Sur l'axe fileté 10 est monté un curseur 53 qui est en prise avec la tête du piston 5 de la seringue 4.

## Revendications

1. Dispositif d'injection électrique comprenant une crosse bâti (1), une seringue (4) qui repose sur la face supérieure horizontale de la crosse bâti (1) et est maintenue dans la partie avant par son embout qui repose sur un étrier (3) et dans la partie arrière par la tête du piston (5) de la seringue (4) qui est logée dans un curseur mobile (6) actionné par un moteur électrique (17) qui transmet la poussée pour déclencher la pénétration de l'aiguille (31) et l'injection du liquide, une mire (24) stabilisatrice de la peau qui guide et prédétermine le degré de pénétration de l'aiguille (31) par un règlage caractérisé par le fait
que la mire stabilisatrice (24) est amovible, qu'un moyen de maintien (34) faisant office de ressort assure son maintien dans une vis creuse micrométrique (46) elle-même logée dans un cylindre mobile (56), ledit cylindre mobile (56) comportant un doigt mobile (40) qui agit sur un moyen faisant office de clapet (35) disposé au niveau de l'aiguille d'injection (4).

2. Dispositif selon la revendication 1 caractérisé par le fait
que la tête du piston (5) de la seringue (4) est en contact avec la tête (9) d'un piston situé dans le curseur mobile (6), le piston (7) est pourvu d'un ressort (8) monté coaxialement à la tige du piston, ledit ressort (8) est logé dans le curseur mobile (6), l'ensemble curseur (6) et piston (7) est actionné par le moteur électrique (17) qui agit sur un axe fileté (10) sur lequel est monté le corps du curseur (6) qui forme à ce niveau un écrou de manoeuvre (45).

3. Dispositif selon la revendication 2 caractérisé par le fait
que la tête (9) du piston (7) est en prise par l'écrou de manoeuvre 45 avec un axe fileté (10) dont une extrémité (11) repose sur un palier (12) situé dans la crosse bâti (1) et dont l'autre extrémité (13) se termine par un pignon denté (14) qui vient engrener sur un pignon denté (15) situé à la sortie d'un réducteur disposé dans le prolongement du moteur électrique (17).

4. Dispositif selon la revendication 1 caractérisé par le fait
que dans le mode de réalisation électrique et électronique, le moteur (50) peut être pas à pas ; le moteur (50) engrène, par son axe et sa roue dentée (51), sur une autre roue (52) en prise directe avec l'axe fileté (10) ; sur l'axe fileté (10) est monté un curseur (53) qui est en prise avec la tête du piston (5) de la seringue (4).

5. Dispositif selon la revendication 3 caractérisé par le fait
qu'un commutateur de fonctionnement (18) est disposé dans le prolongement de l'axe de la chambre 27 et du cylindre 46 qui logent la mire stabilisatrice, ce commutateur de fonctionnement (18) assure le déclenchement du fonctionnement en automatique, continu ou simultané, par action de la mire dès que celle-ci est en contact avec la peau ;
- (19) est le contact marche avant ;
- (20) est le contact marche arrière ;
- (21), disposé dans le prolongement de la tête (9) du piston (7), est l'interrupteur avant ;
- (22), solidaire du curseur (6), est l'interrupteur arrière ;
- (23) est le contacteur marche/arrêt.

6. Dispositif selon la revendication 1 caractérisé par le fait
qu'il est pourvu d'un signal (44), qui est un signal sonore, qui est déclenché à chaque injection.

7. Dispositif selon la revendication 1 caractérisé par le fait
que la mire stabilisatrice (24) pénètre par son extrémité (25), dans un orifice (26) de la partie antérieure de la crosse bâti (1) ; cette extrémité (25) est logée dans une vis micrométrique creuse (46) ; ladite vis micrométrique creuse (46) est filetée sur sa face extérieure de manière à pouvoir se déplacer dans une chambre (27) d'un cylindre mobile (56) prévue à cet effet ; ledit cylindre mobile (56) se termine par un axe (28) ; ledit axe (28) pénètre dans un palier (29), solidaire de la crosse bâti (1); un ressort de rappel (54) est disposé coaxialement audit axe (28) entre la chambre (27) et l'extrémité de son axe (28) ; une molette de réglage (30), permet de faire tourner l'axe (28) et la chambre (27) ; ladite chambre (27) du cylindre mobile (56) comporte un filetage dans son corps intérieur ; le filetage correspond au filetage de la face extérieure de la vis micrométrique (46) ; en faisant tourner la molette de réglage (30), l'axe (28) et la chambre (27) tournent tandis que la vis micrométrique (46) se déplace dans ladite chambre (27) du cylindre mobile (56); cette molette de réglage (30) permet ainsi de régler la profondeur d'entrée de l'extrémité (25) de la mire (24) et de régler ainsi le degré de pénétration de l'aiguille (31) dans la peau.

8. Dispositif selon l'une quelconque des revendications 1 ou 7 caractérisé par le fait
que la mire stabilisatrice (24) se termine par son extrémité (25) qui est cruciforme et qui pénétre dans le corps du cylindre (46) qui est fendu selon son axe longitudinal et uniquement dans sa partie antérieure tandis qu'un moyen de maintien (34) est mis en place autour du corps du cylindre pour faire office de ressort de fixation.

9. Dispositif selon la revendication 8 caractérisé par le fait
que le moyen de maintien (34) est un anneau de maintien qui est mis en place autour du corps de la vis micrométrique creuse (46).

10. Dispositif selon la revendication 1 caractérisé par le fait
qu'il est pourvu d'un moyen (35) faisant office de clapet disposé au niveau de l'aiguille d'injection (4), ledit clapet (35) étant associé à un moyen faisant office de détecteur de pénétration (39) de l'aiguille (31) dans la peau.

11. Dispositif selon la revendication 10 caractérisé par le fait
qu'un corps du raccord pour clapet (36) est disposé entre l'embout (37) de fixation de l'aiguille (31) et l'embout (38) de la seringue où s'emboîte habituellement l'aiguille (31).

12. Dispositif selon la revendication 11 caractérisé par le fait
que le corps du raccord pour clapet (36) est muni d'un moyen faisant office de levier (39) qui est actionné par un doigt mobile (40) ; ledit doigt mobile (40) est vissé sur l'extrémité antérieure de la chambre (27) du cylindre mobile (56); la vis micrométrique creuse (46) peut également être filetée à ce niveau et comporter une butée d'arrêt (48) ; le doigt mobile (40) est extérieur tandis que son corps est cylindrique pour s'emmancher entre la chambre (27) du cylindre mobile (56) et la vis micrométrique (46) ; ledit corps du raccord pour clapet (36) est donc asservi par un moyen qui fait office de détecteur de pénétration de l'aiguille (31) dans la peau ; dès que l'aiguille (31) est retirée de la peau, le clapet (35) ferme l'orifice du corps du raccord pour clapet (36) qui autorise le passage de liquide de la seringue (4) vers l'aiguille (31).

13. Dispositif selon l'une quelconque des revendications 11 ou 12 caractérisé par le fait
que le moyen faisant office de détecteur de pénétration de l'aiguille (31) dans la peau est un levier (39), ledit levier (39) agit au niveau de la paroi (42) du corps du raccord pour clapet (36) ; ladite paroi (42) est, à ce niveau, légèrement plus mince ; ledit levier (39) agit donc directement sur le clapet (35) qui est monté libre dans la chambre (47) ce qui autorise un basculement qui dégage ledit clapet (35) de l'orifice (41) qui autorise la circulation du liquide entre la seringue (4) et l'aiguille (31).

14. Dispositif selon l'une quelconque des revendications 11, 12 ou 13 caractérisé par le fait
que la chambre (47) du corps du raccord pour clapet (36) peut comporter des ergots (43) qui permettent de faire entrer en force le clapet (35) dans ladite chambre (47), mais éviter que celui-ci ne puisse sortir.

15. Dispositif selon la revendication 10 caractérisé par le fait
que le clapet (35) a une forme en tronc de cône.

## Claims

1. An electric injection device comprising a housing (1) and a syringe (4) resting on the horizontal top side of the housing (1) and being held in the front part by its spout resting on a shackle (3) and in the rear part by the head of a piston (5) of the syringe (4), said head being located in a movable slide (6) actuated by an electric motor (17) and transmitting the thrust for releasing the penetration of a needle (31) and the injection of liquid, and a probe (24) stabilizing the skin, guiding the needle 31, and predetermining its degree of penetration by means of a control, characterized in that the stabilizing probe (24) is removable, a holding means (34) which serves as a spring warranting its support in a hollow micrometer screw (46) which, in turn, is accommodated in a movable cylinder (56), said movable cylinder (56) having a movable finger (40) which acts on means serving as a valve (35) which is situated at the level of the injection needle (31).

2. Device according to claim 1, characterized in that the head of the piston (5) of the syringe (4) is in contact with a head (9) of a piston (7) which is disposed in the movable slide (6); a piston (7) has a spring (8) which is mounted coaxially with respect to a rod of the piston; the spring (8) is located in the movable slide (6); and the slide-and-piston unit (6,7) is actuated by the electric motor (17) acting on a threaded shaft (10) on which the body of the slide (6) is mounted, which at this level forms an actuating nut (45).

3. Device according to claim 2, characterized in that the head (9) of the piston (7) is in engagement via the actuating nut (45) with the threaded shaft (10) whose one end (11) rests in a bearing (12) in the housing (1), and whose other end (13) terminates in a pinion (14) being engaged with a pinion (15) at the exit of a reduction gear arranged in the longitudinal extension of the electric motor (17).

4. Device according to claim 1, characterized in that in the electric and electronic embodiment the motor (5) can be a stepping motor; the motor (5) is engaged by its shaft and its gear (51) with another gear (52) which is in direct engagement with the threaded shaft (10); and on the threaded shaft (1) a slide (53) is mounted which is engaged with the head of the piston (5) of the syringe (4).

5. Device according to claim 3, characterized in that a function switch (18) is disposed in the extension of the axis of the chamber (27) and of the cylinder (46) housing the stabilizing probe, the function switch (18) warranting the release of the automatic, continuous, or simultaneous function by the action of the probe as soon as it contacts the skin;
- (19) is the contact for forward movement;
- (20) is the contact for rearward movement;
- (21) is the interruptor forward, which is disposed in the elongation of the head (9) of the piston (7);
- (22) is the interruptor backward connected to the slide (6);
- (23) is the switch for movement and stop.

6. Device according to claim 1, characterized in that a signal means (44) is provided which is an acoustic signal device and is released at each injection.

7. Device according to claim 1, characterized in that the stabilizing probe (24) penetrates with its end (25) into an opening (26) of the front part of the case (1); said end (25) is located in a hollow micrometer screw (46); said micrometer screw (46) is threaded on its outer surface, so that it can slide in a chamber (27) of a movable cylinder (56) provided for this purpose; said movable cylinder (56) is terminated by a shaft (28); said shaft (28) penetrates into a bearing (29) integral with the case (1); a reset spring (54) is diaposed coaxially with respect to the shaft (28) between the chamber (27 and the end of its shaft (28); an adjusting wheel (3) can rotate the shaft (28 and the chamber (27); said chamber (27) of the movable cylinder (56) is internally threaded; the threads correspond to the threads of the outer surface of the micrometer screw (46); by rotating the adjusting wheel (30) the shaft (28) and the chamber (27) rotate, while the micrometer screw (46) shifts in said chamber (27 of the movable cylinder (56); and said adjusting wheel (30) permits in this way an adjustment of the penetration depth of the end (25) of the probe (24) and in this way of the degree of penetration of the needle (31) into the skin.

8. Device according to any one of the claims 1 or 7, characterized in that the stabilizing probe (24) is terminated by its end (25), which is cruciform and penetrates into the body of the cylinder (46) which is slotted along its longitudinal axis and only in its front part; and a holding means (34) is provided around the body of the cylinder and serves as fixing means.

9. Device according to claim 8, characterized in that the holding means (34) is a holding ring which is provided around the body of the hollow micrometer screw (46).

10. Device according to claim 1, characterized in that a means (35) is provided serving as valve disposed at the level of the injection needle (31), the valve (35) being associated to a means serving as detector (39) for penetration of the needle (31) into the skin.

11. Device according to claim 10, characterized in that a valve connecting member (36) is disposed between the spout (37) for fixing the needle (31) and the spout (38) of the syringe where usually the needle (31) is mounted.

12. Device according to claim 11, characterized in that the valve connecting member (36) is provided with a means (39) serving as a lever and being actuated by a movable finger (40); said movable finger (40) is screwed onto the forward end of the chamber (27) of the movable cylinder (56); the hollow micrometer screw (46) can be threaded also at this level and may be provided with a stop (48); the movable finger (40) is disposed on the outside, while its body is cylindrical in order to be accommodated between the chamber (27) of the movable cylinder (56) and the micrometer screw (46); the valve connecting member (34) is thus controlled by a means serving as a detector the penetration of the needle (31) into the skin; and as soon as the needle (31) is retracted from the skin the valve (35) closes the opening in the valve connecting member (36) permitting the passage of the liquid from the syringe (4) to the needle (31).

13. Device according to any one of claims 11 or 12, characterized in that the means serving as a detector for penetration of the needle (31) into the skin is a lever (39); the lever (39) acts at the level of the wall (42) of the valve connecting member (36); said wall (42) is slightly thinner at this level; in this way said lever (39) directly acts on the valve (35) which is provided freely in the chamber (47) thereby permitting a tilting motion disengaging the valve (35) from the opening (41) and allowing the flow of liquid between the syringe and the needle (31).

14. Device according to any one of claims 11, 12, or 13, characterized in that the chamber of the valve connecting member (36) may have projections (43) permitting the valve (35) to be pressed into the chamber (47) but preventing the valve from leaving the chamber (47).

15. Device according to claim 10, characterized in that the valve (35) is of frusto-conical shape.

## Patentansprüche

1. Elektrische Injektionsvorrichtung, die ein Gehäuse (1) und eine auf der horizontalen Oberseite des Gehäuses (1) aufliegende Spritze (4) aufweist, wobei die Spritze (1) an der Vorderseite durch ihren Ansatz, der auf einem Bügel (3) aufliegt, und an der Rückseite durch den in einem beweglichen, durch einen Elektromotor (17) angetriebenen Schieber (6) eingefaßten Kolben (5) der Spritze (4) gehalten wird, der den Druck zum Auslösen des Eindringens der Nadel (31) und zur Injektion der Flüssigkeit überträgt, eine Kimme (24) zur Stabilisierung der Haut, die die Nadel (31) führt und ihre Eindringtiefe durch eine Einstellmöglichkeit vorbestimmt, dadurch gekennzeichnet, daß
die Stabilisierkimme (24) austauschbar ist, eine als Feder dienende Haltevorrichtung (34) ihre Verankerung in einer hohlen Mikrometerschraube (46) sicherstellt, die ihrerseits in einem beweglichen Zylinder (56) gefaßt ist, wobei der bewegliche Zylinder (56) einen beweglichen Finger (40) aufweist, der auf eine als Ventil wirkende, in der Höhe der Injektionsnadel (4, muß wohl 31 sein, A.d.Ü.) angebrachte Vorrichtung (35) einwirkt.

2. Vorrichtung nach Anspruch 1, dadurch gekennzeichnet, daß der Kopf des Kolbens (5) der Spritze (4) in Kontakt steht mit dem Kopf (9) eines in dem beweglichen Schieber (6) angebrachten Kolbens, der Kolben (7) mit einer koaxial zur Kolbenstange angebrachten Feder (8) versehen ist; die Feder (8) in dem beweglichen Schieber (6) eingefaßt ist, das System von Schieber (6) und Kolben (7) von dem Elektromotor (17) angetrieben wird, der auf eine Gewindeachse (10) wirkt, auf der der Körper des Schiebers (6) angebracht ist, der auf dieser Höhe eine Verschiebemutter (45 ) bildet.

3. Vorrichtung nach Anspruch 2, dadurch gekennzeichnet, daß der Kopf (9) des Kolbens (7) über die Verschiebemutter (45) mit einer Gewindeachse (10) kraftschlüssig verbunden ist, deren eines Ende (11) in einem im Gehäuse (1) angebrachten Lager (12) liegt und deren anderes Ende (13) in ein Ritzel (14) ausläuft, das in ein Ritzel (15) eingreift, das sich am Ausgang eines in der Verlängerung des Elektromotors (17) angebrachten Reduziergetriebes befindet.

4. Vorrichtung nach Anspruch 1, dadurch gekennzeichnet, daß in der elektrischen und elektronischen Ausführung der Motor (50) ein Schrittmotor sein kann; der Motor (50) greift über seine Achse und sein Zahnrad (51) in ein anderes, direkt kraftschlüssig mit der Gewindeachse (10) verbundenes Zahnrad (52) ein; auf der Gewindeachse (10) ist ein Schieber (53) angebracht, der mit dem Kopf des Kolbens (5) der Spritze (4) kraftschlüssig verbunden ist.

5. Vorrichtung nach Anspruch 3, dadurch gekennzeichnet, daß ein Funktionsumschalter (18) in der Verlängerung der Achse der Kammer (27) und des Zylinders (46), die die Stabilisierkimme einfassen, angebracht ist; dieser Funktionsumschalter bewirkt das Auslösen des automatischen, kontinuierlichen oder simultanen Betriebs, durch Wirkung der Kimme, sobald diese in Kontakt mit der Haut tritt; - (19) ist der Kontakt Vorwärtslauf;
- (20) ist der Kontakt Rückwärtslauf;
- (21), in der Verlängerung des Kopfs (9) des Kolbens (7) angebracht, ist der Unterbrecher vorwärts;
- (22), mit dem Schieber (6) einteilig verbunden, ist der Unterbrecher rückwärts;
- (23) ist der Kontaktschalter ein/aus.

6. Vorrichtung nach Anspruch 1, dadurch gekennzeichnet, daß sie mit einem Signal (44), das ein Tonsignal ist, versehen ist, das bei jeder Injektion ausgelöst wird.

7. Vorrichtung nach Anspruch 1, dadurch gekennzeichnet, daß die Stabilisierkimme (24) mit ihrem Ende (25) in eine Öffnung (26) an der Vorderseite des Gehäuses (1) eindringt; dieses Ende (25) ist in einer hohlen Mikrometerschraube (46) gefaßt; die Mikrometerschraube (46) ist auf der Außenseite mit einem Gewinde versehen, um sich in einer hierfür vorgesehenen Kammer (27) eines beweglichen Zylinders (56) fortbewegen zu können; der bewegliche Zylinder (56) endet in einer Achse (28); die Achse (28) dringt in ein mit dem Gehäuse (1) verbundenes Lager (29) ein; eine Rückholfeder (54) ist koaxial zu der Achse (28) zwischen der Kammer (27) und dem Ende seiner Achse (28) angebracht; ein Einstellrad (30) ermöglicht die Drehung der Achse (28) und der Kammer (27); die Kammer (27) des beweglichen Zylinders (56) weist ein Innnengewinde auf; das Innengewinde entspricht dem Außengewinde der Mikrometerschraube (46); durch Drehen des Einstellrades (30) drehen sich die Achse (28) und die Kammer (27), während die Mikrometerschraube (46) sich in der Kammer (27) des beweglichen Zylinders (56) verschiebt; so ermöglicht dieses Einstellrad (30) die Regelung der Befestigungstiefe des Endes (25) der Kimme (24) und folglich die Regelung der Eindringtiefe der Nadel (31) in die Haut.

8. Vorrichtung nach einem der Ansprüche 1 oder 7, dadurch gekennzeichnet, daß die Stabilisierkimme (24) in ein Endstück (25) ausläuft, das kreuzförmig ist und in den Körper des Zylinders (46) eindringt, der entlang seiner Längsachse und nur im vorderen Teil gespalten ist, während eine Haltevorrichtung (34) rings um den Zylinderkörper angebracht ist um als Befestigungsfeder zu dienen.

9. Vorrichtung nach Anspruch 8, dadurch gekennzeichnet, daß die Haltevorrichtung (34) ein Haltering ist, der um den Körper der hohlen Mikrometerschraube (46) herumgelegt ist.

10. Vorrichtung nach Anspruch 1, dadurch gekennzeichnet, daß sie mit einer als Ventil dienenden Einrichtung (35) in Höhe der Injektionsnadel (4)(muß wohl 31 sein, A.d.Ü.) versehen ist, wobei das Ventil (35) mit einer als Detektor für das Eindringen der Nadel (31) in die Haut dienenden Einrichtung (39) funktionsmäßig verbunden ist.

11. Vorrichtung nach Anspruch 10, dadurch gekennzeichnet, daß ein Ventil-Verbindungskörper (36) zwischen dem Befestigungsansatz (37) der Nadel (31) und dem Ansatz (38) der Spritze, wo üblicherweise die Nadel (31) aufgesteckt wird, angebracht ist.

12. Vorrichtung nach Anspruch 11, dadurch gekennzeichnet, daß der Ventil-Verbindungskörper (36) mit einer als Hebel dienenden Einrichtung (39) versehen ist, die durch einen beweglichen Finger (40) betätigt wird; dieser bewegliche Finger (40) ist auf das Vorderende der Kammer (27) des beweglichen Zylinders (56) aufgeschraubt; die hohle Mikrometerschraube (46) kann auch auf dieser Höhe mit Gewinde versehen sein und einen Halteanschlag (48) aufweisen; der bewegliche Finger (40) liegt außen, während sein Körper zylindrisch ist, um sich zwischen die Kammer (27) des beweglichen Zylinders (56) und die Mikrometerschraube (46) einzufügen; der Ventil-Verbindungskörper (36) wird also gesteuert durch eine Einrichtung, die als Detektor für das Eindringen der Nadel (31) in die Haut dient; sobald die Nadel (31) aus der Haut gezogen wird, schließt das Ventil (35) die Öffnung im Ventil-Verbindungskörper (36), die den Durchgang der Flüssigkeit von der Spritze (4) zur Nadel (31) ermöglicht.

13. Vorrichtung nach einem der Ansprüche 11 oder 12, dadurch gekennzeichnet, daß die als Detektor für das Eindringen der Nadel (31) in die Haut dienende Einrichtung ein Hebel (39) ist, der Hebel (39) wirkt in Höhe der Wand (42) des Ventil-Verbindungskörpers (36); die Wand (42) ist auf dieser Höhe geringfügig dünner; der Hebel (39) wirkt so direkt auf das Ventil (35), das frei in der Kammer (47) angebracht ist, wodurch eine Klappbewegung ermöglicht wird, die das Ventil (35) von der Öffnung (41) löst und den Strom der Flüssigkeit zwischen der Spritze (4) und der Nadel (31) ermöglicht.

14. Vorrichtung nach einem der Ansprüche 11, 12 oder 13, dadurch gekennzeichnet, daß die Kammer (47) des Ventil-Verbindungskörpers (36) Vorsprünge (43) aufweisen kann, die es ermöglichen, das Ventil (35) in die Kammer (47) hinein-zudrücken, es jedoch verhindern, daß es die Kammer (47) verläßt.

15. Vorrichtung nach Anspruch 10, dadurch gekennzeichnet, daß das Ventil (35) die Form eines Kegelstumpfs hat.
